# EUROPEAN PATENT APPLICATION

(11) **EP 2 596 758 A1**
(43) Date of publication of application: **29.05.2013**
(21) Application number: 11190510.5
(22) Date of filing: 24.11.2011
(51) Int. Cl.: A61B 17/86, A61F 2/08

(54) **Bone screw**

(71) Applicant: Sysorb GmbH, 4052 Basel (CH)
(72) Inventor: Stähelin, Andreas Christoph, 4052 Basel (CH)
(74) Representative: Bohest AG

(57) **Abstract**

In one aspect the invention relates to a bone screw (1; 11; 21) comprising an external thread (2, 12; 22) extending about a longitudinal axis (L) of the screw, and an inner engagement contour (8; 18; 28) for receiving a complementary engagement contour (211) of a screwdriver (210), wherein in at least a portion of the screw (1; 11; 21) the thread (2; 12; 22) is formed by the thread crest (3; 13; 23) only. In another aspect the invention relates to a suture anchor comprising a bone screw (11; 21) and a suture (107; 207) affixed to the distal end (16; 26) of the screw (11; 21). Yet in another aspect the invention relates to a method for fixing a suture anchor in a bone.

## Description

The invention generally relates to a bone screw in accordance with the preamble of the independent patent claim.

### BACKGROUND OF THE INVENTION

When soft tissue, such as tendons and ligaments, are torn away from the bone, surgery may be required to repair the tear. Various devices have been suggested to anchor tissue to a bone. These devices are usually attached to the bone through the use of open surgery or preferably through arthroscopic surgical techniques.

Known devices used for attaching soft tissue to the bone include sutures, staples, wedges, cements, screws and anchors. Various types of bone screws are known in the art.

For example EP-A-0674880 discloses a biodegradable interference screw with an elongated screw body which extends between the proximal and distal end of the screw, wherein an outer surface of the screw body is provided with an outer thread coaxial to a longitudinal axis, which thread is intended for guiding and holding the screw in the bone material of a patient. In the screw body an elongated channel coaxial to the longitudinal axis is provided, which at the proximal end is open for accommodation of a shaft of a screwdriver intended for tightening the screw.

Commercially available bone screws are for example known under the tradenames Biosure^{™}, Mega Fix^{™} or Sysorb^{™}.

Known materials for biodegradable bone screws, in particular for biodegradable interference screws, are polylactides (e.g. Poly-L-lactic acid, PLLA), polyglycolides (e.g. Polyglycolic acid, PGA) or copolymers thereof, which are exceptionally formable, sterilizable and absorbable materials. EP-A-0502698 discloses Poly(L-Lactide) as a particulary suitable biodegradable, water-soluble and non-toxic material for a bone screw.

Known biocompatible materials for suture anchors, which are usually intended to be not biodegradable, are metals or alloys (such as for example titan, titan alloys, stainless steel or stainless steel alloys), polymers (such as for example polyethylene etherketones, PEEK), or ceramics.

Another category of know materials for bone screws are so-called biocomposites, which comprise a biocompatible inorganic component (e.g. calcium phosphate) and an organic component (e.g. a biodegradable polymer). A biocomposite may for example comprise 30% of a biphasic calcium phosphate and 70% of a polylactide.

Required properties for bone screws are *inter alia* a sufficient hardness for screwing-in of the screw, a sufficient tensile strength in particular for holding a suture in place when the screw is used as a suture anchor, and a sufficient compressive strength for displacing bone material upon screwing-in of the screw as well as for ensuring firm hold of the screw in the bone and fixation of the soft tissue, such as tendons and ligaments, to the bone.

Although a varity of bone screws are known in the art, there are still aspects which can be improved. It is thus an objective of the present invention to provide an improved bone screw which is useful as an interference screw. It is a further objective of the present invention to provide an improved suture anchor based on said bone screw.

### SUMMARY OF THE INVENTION

This object is achieved by a bone screw as is characterized by the features of the independent patent claim. Advantageous embodiments of the bone screw according to the invention are apparent from the features of the dependent patent claims.

In one aspect the invention relates to a bone screw comprising an external thread extending about a longitudinal axis of the screw, the external thread comprising a thread crest; an inner engagement contour for receiving a complementary engagement contour of a screwdriver; wherein in at least a portion of the screw the thread is formed by the thread crest only. It is an advantage of said aspect of the invention that, at least in the portion of the screw where the thread is formed by the thread crest only, the bone can grow into the inner parts of the screw, once the screw has been applied to a patient's bone.

In another aspect the bone screw according to the invention further comprises one or more inner elongated ribs extending from a proximal end towards a distal end of the screw and connecting the thread crest over the length of the elongated ribs. Thereby the overall stability of the bone screw is increased in said rib portion, in particular with regard to compressive strength and torsional stiffness of the screw. In a preferred aspect of said screw the one or more inner elongated ribs are extending from the proximal end over at least one third of the length of the screw.

In yet another aspect the bone screw according to the invention further comprises an intermediate portion, which extends from the end of the portion comprising the one or more inner elongated ribs to three quarters of the length of the screw, wherein in said intermediate portion the thread is formed by the thread crest only. The intermediate portion provides for axial compressibility of the screw (i.e. in the direction of the longitudinal axis), as well as it allows the bone to grow into the inner parts of the screw, once the screw has been applied to a patient's bone.

In yet another aspect of the invention, the portion of the screw in which the thread is formed by the thread crest only extends from the proximal end of the screw towards the distal end of the screw over at least three quarters of the length of the screw. The axial compressibility of the screw (i.e. in the direction of the longitudinal axis) is thereby enhanced, as well as is the ability of the screw to allow bone to grow into the inner parts of the screw, once the screw has been applied to a patient's bone.

In yet another aspect the bone screw according to the invention further comprises a distal portion extending from the distal end towards the proximal end of the screw over one quarter of the length of the screw, wherein the external thread comprises a thread crest and a thread groove. The distal portion of the screw improves the ability of the screw for displacing and/or compressing bone material upon screwing-in of the screw. The resistance to compressive forces exerted by the bone upon screwing-in, as well as the ability of the external thread of the screw to cut (or tap) a corresponding thread in the bone are thereby improved.

In a preferred aspect the screw is tapered, which improves the above named abilities of the screw even further. For screwing-in it may thus be dispensed with the preliminary cutting of a hole or thread into the bone even when the screw is applied to hard bone, which considerably simplifies the operation method. The tapered screw generally also improves the guidance of the screw upon screwing-in, independently from whether a hole or thread has been pre-cut into the bone.

In yet another aspect of the invention the inner engagement contour extends from the proximal end of the screw substantially to the distal end of the screw. The term "substantially to the distal end of the screw" refers to at least about 80% of the length of the screw, preferably at least about 85% of the length of the screw and in particular about seven eighth of the length of the screw. In an alternative aspect of the invention the inner engagement contour extends through the entire screw from the proximal end to the distal end of the screw.

For screwing-in of the screw, the screwdriver introduced into the engagement contour of the screw resembles a screw body and thus, together with the screw, provides for compressive strength and torsional stiffness of the assembly comprising the screw and the screwdriver. Further, the screwdriver engaged with the inner engagement contour of the screw over a substantial part of the length of the screw, in particular in the portion where the screw is formed by the thread crest only, supports the external thread for compressive strength of the screw, for displacing and/or compressing bone material upon screwing-in of the screw. Additionally, the hard tip of the screwdriver extending through the distal end of the screw provides for sufficient hardness of the distal end of the assembly comprising the screw and the screwdriver for screwing-in of the screw.

In yet another aspect of the invention the inner engagement contour is at least partly formed in the inner wall of the thread crest and comprises two or more recesses which are inclined in a direction of rotation for screwing-in of the screw, each recess comprising a leading flank and a trailing flank, with the leading flank being shorter than the trailing flank. As the main forces are usually exerted upon screwing-in of a bone screw according to the invention, an inclination of the recesses in the direction of rotation for screwing-in improves the ability of the screw for taking on and transferring the main forces, for increasing purchase and for avoiding excessive expansive forces to the screw and to the bone. A screw of the present invention thus generally tolerates greater torques compared to conventional screws. For screwing-in it may thus be dispensed with the preliminary cutting of a hole or thread into the bone even when the screw is applied to hard bone, which considerably simplifies the operation method.

There are further advantages of the screw according to the present invention in which the inner engagement contour is at least partly formed in the inner wall of the thread crest:

Firstly, for a screwdriver with a given diameter of the complementary engagement contour on the shaft of the screwdriver, a screw of the invention can have a smaller outer (overall) diameter compared to a conventional screw. This is due to the fact that the outer (overall) diameter of the screw is defined by the thread crest, whereas the diameter of the screwdriver (i.e. of the complementary engagement contour on the shaft of the screwdriver) is defined by the maximum diameter of the inner engagement contour available in the screw. Thus, the fact that the screw according to the present invention is devoid of a screw body as generally referred to in the prior art, allows for a smaller outer (overall) diameters of the screw relative to the diameter of the screwdriver.

Secondly, for a screw with a given outer (overall) diameter, a screwdriver can have a larger diameter (i.e. the diameter of the complementary engagement contour on the shaft of the screwdriver) compared to a screwdriver used for a conventional screw with the same outer (overall) diameter. This is due to the fact that the inner engagement contour available in the screw can not only extend to an inner wall of a body of the screw (which for a conventional screw on the outside carries the thread crest), but can extend at least partly into the inner wall of the thread crest itself. Thus, the fact that the screw according to the present invention is devoid of a screw body as generally referred to in the prior art, allows for a larger diameter of the screwdriver relative to the outer (overall) diameter of the screw.

On the one hand this makes screws available which, based on the same materials, are less prone to breakage upon screwing-in, as a larger diameter of a screwdriver generally better distributes and transfers forces to the screw. Furthermore, greater torques can be applied to a screw with a larger diameter of a screwdriver, which may allow the surgeon to dispense with the preliminary cutting of a hole or thread into the bone.

On the other hand this makes screws available which, based on the same tools currently used for screwing-in, may have a smaller diameter and thus may increase the flexibility of the surgeon to selected a suitable screw for a particular patient. Furthermore, smaller screws are generally degraded (absorbed) faster in a patient's bone, which thus may allow the surgeon to select a screw in accordance with the anticipated healing time for the fixated tissue of a particular patient.

In a preferred aspect the inner engagement contour comprises three to eight recesses, in particular six recesses.

In yet another aspect of the invention the screw further comprises an opening through the distal end face of the screw. The opening allows the tip of a screwdriver to extend through the distal end of the screw and thus providing a hard tip to the distal end of the assembly comprising the screw and the screwdriver for screwing-in of the screw. The hard tip improves the ability of the screw for displacing and/or compressing bone material upon screwing-in of the screw. The resistance to compressive forces exerted by the bone upon screwing-in, as well as the ability to cut (or tap) a thread into the bone are improved.

In another aspect the invention relates to a suture anchor comprising a bone screw as described above and further comprising a suture affixed to the distal end of the screw.

In one aspect thereof the suture first extends along the outer contour of the screw along the entire length of the screw from the proximal end to the distal end of the screw and then on the same or on the other side of the screw extends back to and beyond the proximal end of the screw.

In another aspect thereof the suture first extends along the inside of the screw along the entire length of the screw, passes through an opening in the distal end face of the screw, and then extends along the outer contour of the screw over the entire length of the screw from the distal end to and beyond the proximal end of the screw.

In yet another aspect thereof the suture extends along the inside of the screw along the entire length of the screw, passes through an opening in the distal end face of the screw, then extends along a portion of the outer contour of the screw over the thread crest and thread groove of the external thread in the distal portion, then passes into the inside of the screw through an opening in the portion of the screw in which the thread is formed by the thread crest only, and finally extends again along the inside of the screw over the entire length of the screw out of the proximal end of the screw.

In yet another aspect the invention relates to a method for fixing a screw in a bone, wherein in a first step a screw as described above is screwed into a bone by means of a screwdriver. For screwing-in of the screw, the screwdriver introduced into the engagement contour of the screw resembles a screw body and thus, together with the screw, provides for compressive strength and torsional stiffness of the assembly comprising the screw and the screwdriver. Further, the screwdriver engaged with the inner engagement contour of the screw over a substantial part of the length of the screw, in particular in the portion where the screw is formed by the thread crest only, supports the external thread for compressive strength of the screw, for displacing and/or compressing bone material upon screwing-in of the screw. Additionally, the hard tip of the screwdriver extending through the distal end of the screw provides for sufficient hardness of the distal end of the assembly comprising the screw and the screwdriver for screwing-in of the screw.

In still another aspect the invention relates to a method for fixing a suture anchor in a bone, wherein in a first step a suture anchor as described above is screwed into a bone by means of a screwdriver, and wherein in a second step the distal end of the anchor is tilted and/or at least partly compressed by means of pulling the suture extending beyond the proximal end of the screw. After the first step and before the second step the screwdriver is retracted. The suture affixed to the distal end of the screw, upon pulling the suture into the direction of the proximal end, exerts forces to the screw in the distal portion. Due to compressibility (i.e. flexibility) of the portion in which the thread is formed by the thread crest only, the distal portion of the screw is displaced. The displacement is either in axial direction (i.e. compression of the screw in the portion in which the thread is formed by the thread crest only) and/or in an angular direction relative to the longitudinal axis (i.e. tilting the distal portion and/or part of the portion in which the thread is formed by the thread crest only). Either way, the screw is jammed in the bone, thus further securing the firm hold of the anchor in the bone.

The screws according to the invention are worked to the desired shape in a known manner. For example, if polymeric materials are used for the the bone screw, known methods for forming the screw are injection molding, hot forming, hot press molding and similar processes at suitable temperatures, preferably in the range between room temperature and 100°C. For metals or alloys known methods for forming the screw are casting, forging, drilling, milling, turning, etc., for ceramics a known method is sintering.

### BRIEF DESCRIPTION OF THE DRAWINGS

Exemplary embodiments of the invention are explained in greater detail hereinbelow with reference to the drawings, in which:
FIG. 1A shows one embodiment of a screw according to the invention, in a perspective side view;
FIG. 1B and FIG. 1C show top views of the screw of FIG. 1A, in order to illustrate the design of the inner engagement contour;
FIG. 1D and 1E show the screw of FIG. 1A in two longitudinal sections at different rotational angles as shown in FIGS. 1B and 1C, whith a shaft of a screwdriver inserted therein being indicated;
FIG. 2 shows an embodiment of a screwdriver suitable for screws according to the invention, including an enlarged view on the distal end of the shaft of the screwdriver;
FIG. 3A shows another embodiment of a screw according to the invention, in a perspective side view;
FIG. 3B and FIG. 3C show top views of the screw of FIG. 3A, in order to illustrate the design of the inner engagement contour;
FIG. 3D and 3E show the screw of FIG. 3A in two longitudinal sections at different rotational angles as shown in FIGS. 3B and 3C, whith a shaft of a screwdriver inserted therein being indicated;
FIG. 4A shows yet another embodiment of a screw according to the invention, in a perspective side view;
FIG. 4B and FIG. 4C show top views of the screw of FIG. 4A, in order to illustrate the design of the inner engagement contour;
FIG. 4D and 4E show the screw of FIG. 4A in two longitudinal sections at different rotational angles as shown in FIGS. 4B and 4C, whith a shaft of a screwdriver inserted therein being indicated;
FIG. 5 shows an embodiment of a suture anchor according to the present invention in a perspective side view;
FIG. 6 shows another embodiment of a suture anchor according to the present invention in a perspective side view.

### DETAILED DESCRIPTION OF EMBODIMENTS

Further advantageous aspects will become clear from the following description of illustrative embodiments of the bone screw, as well as of the suture anchor according to the invention, the description being given wherever possible with reference to the drawings.

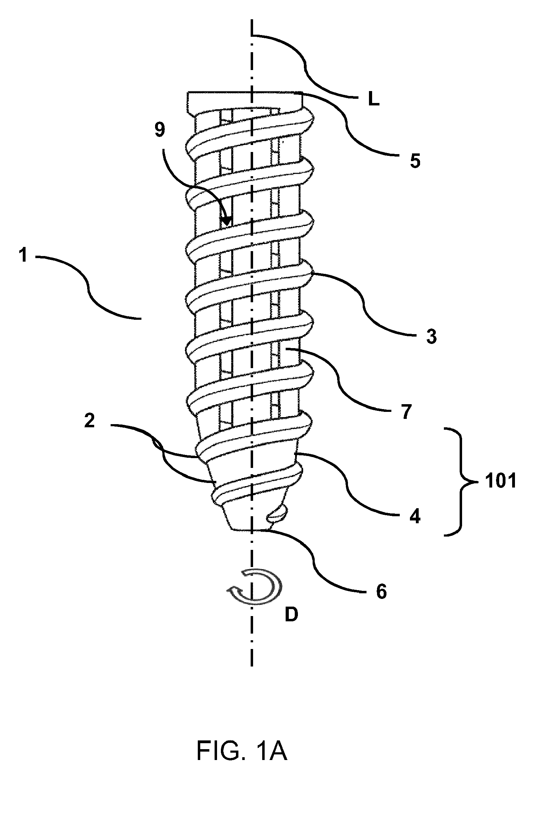

FIG. 1A shows one embodiment of a bone screw according to the invention in a perspective side view. The screw 1 has a proximal end 5 and a distal end 6 and comprises an external thread 2 extending about a longitudinal axis L of the screw 1, with a clockwise direction of rotation D which generally is referred to as the direction of rotation for screwing-in of the screw.

The external thread 2 is configured for guiding and holding the screw in the bone of a patient. Upon screwing-in the distal end 6 of the screw 1 is advancing into the bone. Depending on the condition of the bone as well as depending on the material from which the screw is made, preliminary cutting of a thread or hole in the bone may be required.

Usually, an external thread 2 of a screw comprises a thread crest 3 and a thread groove 4 adjacent to the said thread groove. For the bone screw according to the invention however, the thread 2, in at least a portion of the screw 1, is formed by the thread crest 3 only.

The embodiment of the bone screw according to the invention shown in FIG. 1A further comprises an inner engagement contour for receiving a complementary engagement contour of a screwdriver, which are all not shown in FIG. 1A.

It is to be noted, that the term "complementary contour" or "complementary engagement contour" (of a screwdriver) as used in the present application is defined in that said contour is suitable for drivingly engaging with a respective inner engagement contour of a screw.

Still further the embodiment of FIG. 1A comprises a distal portion 101 extending from the distal end 6 towards the proximal end 5 of the screw over about one quarter of the length of the screw 1, wherein the external thread 2 comprises a thread crest 3 and a thread groove 4. Further, in the embodiment of FIG. 1A, the screw 1 is tapered in the distal portion 101.

In a preferred embodiment of a bone screw according to the invention the thread 2 in the distal portion 101 is formed by the thread crest 3 and a thread groove 4 for at least one convolution, preferably at least two, in particular two convolutions of the thread.

It is to be noted that in the present application each range or portion defined with the term "about" or "at least" also explicitly includes and discloses, as an individual embodiment, the exact value given as the limit of the said range or portion.

Still further the embodiment of FIG. 1A comprises six inner elongated ribs 7 extending from the proximal end 5 towards the distal end 6 of the screw 1. Due to the side view shown in FIG. 1A, only three of the six ribs 7 are visible. The inner elongated ribs 7 are connecting the thread crest 3 over the length of the ribs 7. Further, the inner elongated ribs 7 are partly supporting the thread crest 3, whereas in at least a portion, i.e. in particular in a portion 9 adjacent to each of the inner elongated ribs 7, the thread crest 3 is unsupported. Accordingly, the screw 1 is perforated (or fenestrated) in order to allow bone to grow into the inner parts of the screw, once the screw has been screwed in to a patient's bone.

In a preferred embodiment of a bone screw according to the invention, the one or more inner elongated ribs 7 are supporting and connecting the thread crest 3 for at least one convolution, in particular over six convolutions of the thread.

It is to be noted that the above described embodiment of FIG. 1A of the screw is devoid of a screw body as generally referred to in the prior art. It is further to be noted, that in the above embodiment, although not shown in FIG. 1A, the inner engagement contour is formed at least partly in the inner wall of the thread crest 3, and may optionally as well partly be formed in the inner wall of the elongated ribs 7.

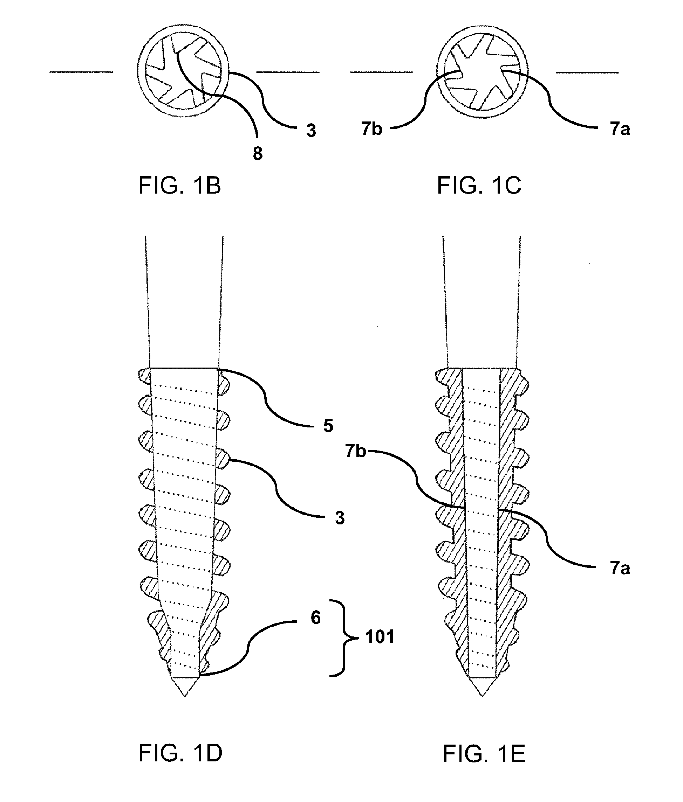

FIG. 1B and FIG. 1C show top views of the screw of FIG. 1A, in order to illustrate the design of the inner engagement contour 8 described in more detail below. The outer circular line of FIG. 1B (as well as of FIG. 1C) represent the thread crest 3 of the screw, whereas the inner contour line, i.e. the six protrusions, in FIG. 1C (as well as of FIG. 1B) represent the elongated ribs, two of which are indicated as 7a and 7b.

In particular in FIG. 1B and FIG. 1C the inner engagement contour 8 is represented in a cross-section of the screw 1, at right angles to the longitudinal axis L, and generally comprises from three to eight recesses between the elongated ribs and alternating with the elongated ribs, two of which are indicated as 7a and 7b. In particular in FIG. 1B and 1C six recesses are indicated, which are all inclined in the direction of rotation D for screwing-in of the screw. Each recess comprises a leading flank and a trailing flank and optionally a base. The leading flank is shorter than the trailing flank and thus, at a certain radial distance from the longitudinal axis, the flanks will intersect or converge at an angle to close the recess. The leading flank is for example inclined at an angle of up to 10 degrees and in FIGS. 1B and 1C in particular at an angle of about 5 degrees. Optionally the recess may be closed by a base, which at a certain radial distance from the longitudinal axis is connecting the flanks, to form a bottom of the recess. In the embodiment of FIG. 1B and 1C the inner engagement contour 8, i.e. the recesses together substantially resemble a sawtooth-like profile.

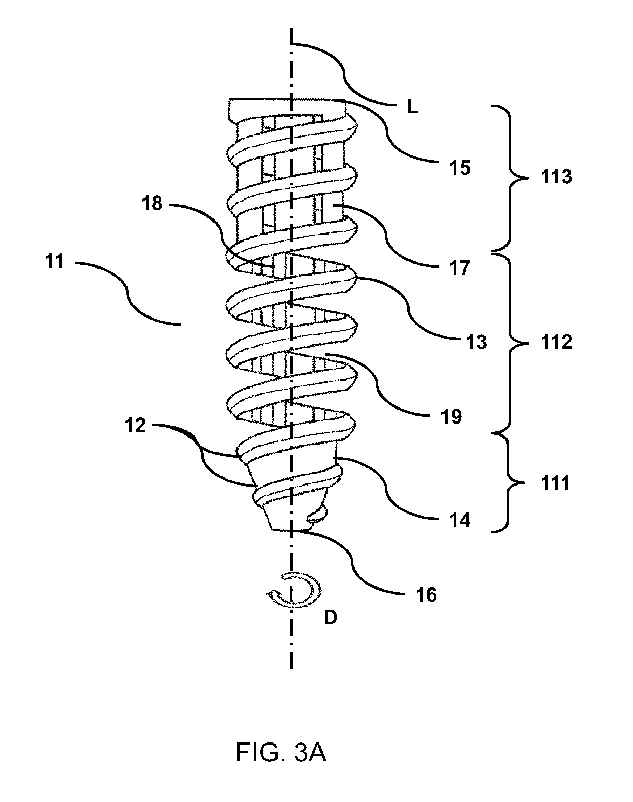

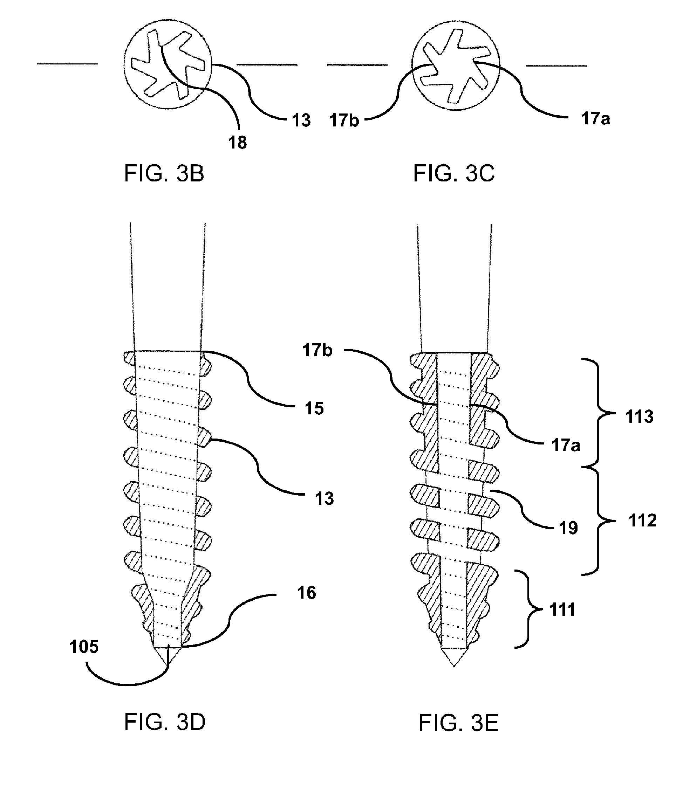

Further suitable engagement contours are described in EP-A-0674880. In particular the engagement contours represented there in FIGS. 3-6, 9, 11, 13 and 15, with leading and trailing flanks (indicated as 24 and 25 in EP-A-0674880) directly intersecting (or converging) and thus closing the recesses (indicated as 23 there), as well as those represented in FIGS. 7, 8, 10, 12, 14 and 16 of EP-A-0674880, with leading and trailing flanks (indicated as 24 and 25 there) closed by a base (indicated as 27 there) connecting the flanks at certain radial distance from the longitudinal axis (indicated as 14 therein), thus forming a bottom of the recesses (indicated as 23 therein), are considered suitable engagement contours. Within the meaning of the present invention, all embodiments of the engagement contour shown in EP-A-0674880 substantially resemble a sawtooth-like profile.

As a consequence of the the above described design, the inner engagement contour of the bone screw of the present invention is rotationally symmetrical about the longitudinal axis L at fixed rotational angles, and is asymmetrical with respect to a diametrical plane including the longitudinal axis L. Rotational symmetry of order n, also called n-fold rotational symmetry, or discrete rotational symmetry of the n^{th} order, with respect to a an axis is defined in that rotation by an angle of 360°/n does not change the object (e.g. for a C₄ symmetrie, i.e. n being 4 a rotation by an angle of 90° does not change the object).

FIGS. ID and 1E show the screw of FIG. 1A in two longitudinal sections, at different rotational angles as shown in FIGS. 1B and 1C. Due to the different rotational angle of the longitudinal sections FIG. 1D shows the unsupported portions of the thread crest 3, whereas the FIG. 1E shows the thread crest 3 supported and connected by two elongated ribs (7a, 7b).

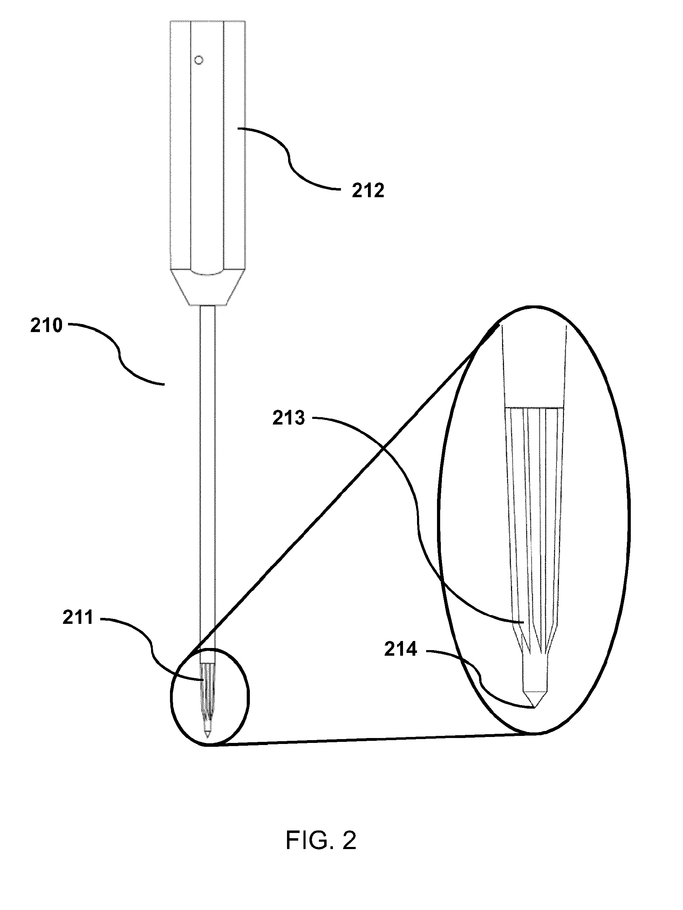

Further, in FIGS. 1D and 1E a shaft 211 of a screwdriver 210 (see FIG. 2) inserted into the screw through the proximal end 5 is indicated, in particular with the tip 214 of the shaft 211 (see FIG. 2) extending through an opening at the distal end 6 of the screw 1.

A screwdriver 210 suitable for a bone screw according to the present invention is shown in FIG. 2. It comprises a shaft 211 with a complementary contour 213 to the inner engagement contour of the screw, and a grip 212. At the proximal end, the screw is open to receive the shaft 211. The shaft 211 of the screwdriver 210 can thus be introduced into the engagement contour of the screw and, in reverse, can be removed therefrom. The blades 213 of the screwdriver are inclined in the direction of rotation D (see FIG. 1A), i.e. the direction of rotation for screwing-in of the screw. The screwdriver 210, with the shaft 211 introduced into the engagement contour of the screw, can thus be operated to screw the screw into the bone.

FIG. 3A shows another embodiment of a bone screw according to the invention in a perspective side view. The screw 11 has a proximal end 15 and a distal end 16. The bone screw 11 comprises an external thread 12 extending about a longitudinal axis L of the screw, with a clockwise direction of rotation D, which generally is referred to as the direction of rotation for screwing-in of the screw. The external thread 12 is configured for guiding and holding the screw in the bone of a patient. Upon screwing-in the distal end 16 of the screw 11 is advancing into the bone. Depending on the condition of the bone as well as depending on the material from which the screw is made, preliminary cutting of a thread or hole in the bone may be required. Usually, an external thread 12 of a screw comprises a thread crest 13 and a thread groove 14 adjacent to the said thread groove. For the bone screw according to the invention however, the thread 12, in at least a portion 112 of the screw 11, is formed by the thread crest 13 only.

The bone screw further comprises an inner engagement contour 18 for receiving a complementary engagement contour of a screwdriver.

Still further the embodiment of FIG. 3A comprises a distal portion 111 extending from the distal end 16 towards the proximal end 15 of the screw over about one quarter of the length of the screw 11, in which the external thread 12 comprises a thread crest 13 and a thread groove 14. In the embodiment of FIG. 3A the screw 11 is tapered in the distal protion 111.

In a preferred embodiment of a bone screw according to the invention the thread 12 in the distal portion 111 is formed by the thread crest 13 and a thread groove 14 for at least one convolution, preferably at least two, in particular two convolutions of the thread.

Still further the embodiment of FIG. 3A comprises six inner elongated ribs 17 extending from the proximal end 15 over at least one third of the length of the screw 11. In particular ribs 17 extend from the proximal end 15 to about one third of the length of the screw. In said portion 113, the inner elongated ribs 17 are partly supporting the thread crest 13, whereas in a portion in axial direction adjacent to each of the inner elongated ribs 17, the thread crest 13 is unsupported. The inner elongated ribs 17 are connecting the thread crest 13 over the length of the ribs 17. Due to the perspective side view shown in FIG. 3A, only three of the six ribs 17 are visible.

In a preferred embodiment of a bone screw according to the invention, the one or more inner elongated ribs 17 are supporting and connecting the thread crest 13 for at least one convolution, in particular three convolutions of the thread.

In the embodiment of FIG. 3A an intermediate portion 112 is defined which extends from the end of the portion 113 comprising the one or more inner elongated ribs 17 to about three quarters of the length of the screw. In said intermediate portion 112 the thread 12 is formed by the thread crest 13 only. In a preferred embodiment of a bone screw according to the invention the thread in the intermediate portion 112 is formed only by the thread crest 13 for of at least one convolution, preferably from two to six convolutions, in particular from three to four convolutions.

Accordingly, the screw in the intermediate portion 112 neither comprises a thread groove nor any ribs, it is open to the inside in order to allow bone to grow into the inner parts of the screw, once the screw has been screwed into a patient's bone. Furthermore, part of the screw is axially compressible in the direction of the longitudinal axis.

Also here it is to be noted that the above described embodiment of the screw is devoid of a screw body as generally referred to in the prior art. It is further to be noted, that in the embodiment shown in FIG. 3A the inner engagement contour 18 is formed at least partly in the inner wall of the thread crest 13, and may although not shown in FIG. 3A optionally as well partly be formed in the inner wall of the elongated ribs 17.

FIG. 3B and FIG. 3C show top views of the screw of FIG. 3A, in order to illustrate the design of the inner engagement contour 18 which is described in more detail above (see description of FIG. 1B and FIG. 1C above). The outer circular line of FIG. 3B (as well as of FIG. 3C) represents the thread crest 13 of the screw, whereas the inner contour line, i.e. the six protrusions represent the elongated ribs, two of which are indicated as 17a and 17b.

FIG. 3D and 3E show the screw of FIG. 3A in two longitudinal sections at different rotational angles of the screw as shown in FIGS. 3B and 3C. Due to the different rotational angle of the longitudinal sections, FIG. 3D shows the unsupported portions of the thread crest 13 in the rib portion 113 as well as in the intermediate portion 112 (see FIG. 3A) of the screw, whereas FIG. 3E in the rib portion 113 of the screw shows the thread crest 13 supported by two elongated ribs (17a, 17b).

Accordingly, the screw shown in FIGS. 3A-E, in the portion where the screw 11 is formed by the thread crest 13 only, is open to the inside in order to allow bone to grow into the inner parts of the screw, once the screw has been screwed into a patient's bone. The opening 19 is provided at a location where in a conventional screw a thread groove would be provided, i.e. adjacent to the thread crest 13 and extending about the longitudinal axis L of the screw 11, and with the same direction of rotation D as the external thread.

In FIGS. 3D and 3E a shaft of a screwdriver inserted into the screw through the proximal end 15 is indicated, in particular with the tip 214 of the shaft 211 (see FIG. 2) extending through an opening 105 at the distal end 16 of the screw 11. Thus, with the screwdriver inserted, the blades 213 (see FIG. 2) of the screwdriver are partly visible from the outside through the opening 19 of the screw.

For screwing-in of the screw, the screwdriver introduced into the engagement contour of the screw resembles a screw body and thus, together with the screw, provides for compressive strength and torsional stiffness of the assembly comprising the screw and the screwdriver.

Further, the screwdriver engaged with the inner engagement contour 18 of the screw 11 over a substantial part of the length of the screw, but in particular in the intermediate portion 112 (see FIG. 3A) where the screw is formed by the thread crest 13 only, supports the external thread for compressive strength of the screw 11, in particular for displacing and/or compressing bone material upon screwing-in of the screw 11. Additionally, the hard tip 214 of the screwdriver 210 (see FIG. 2) extending through the distal end 16 of the screw 11 provides for sufficient hardness of the distal end of the assembly comprising the screw 11 and the screwdriver 210 for screwing-in of the screw.

Furthermore, upon retracting the screwdriver, the screw 11 has sufficient compressive strength for ensuring firm hold of the screw in the bone and for fixation of soft tissue, such as tendons and ligaments, to the bone. Also, the screw alone has sufficient tensile strength for holding a suture in place when the screw is used as a suture anchor.

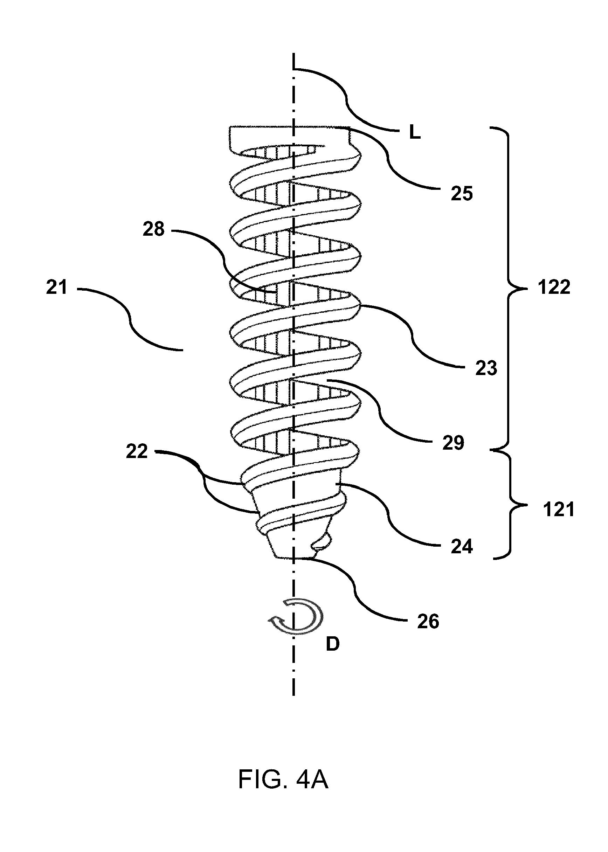

FIG. 4A shows another embodiment of a bone screw according to the invention in a perspective side view. The screw 21 has a proximal end 25 and a distal end 26. The bone screw 21 comprises an external thread 22 extending about a longitudinal axis L of the screw, with a clockwise direction of rotation D, which generally is referred to as the direction of rotation for screwing-in of the screw.

The bone screw further comprises an inner engagement contour 28 for receiving a complementary engagement contour of a screwdriver (see above). In the embodiment of FIG. 4A, the inner engagement contour 28 is formed entirely in the inner wall of the thread crest 23.

Still further the embodiment of FIG. 4A comprises a distal portion 121 extending from the distal end 26 towards the proximal end 25 of the screw 21 over about one quarter of the length of the screw 21, wherein the external thread 22 comprises a thread crest 23 and a thread groove 24. In the embodiment of FIG. 4A the screw 21 is tapered in the distal protion 121.

In a preferred embodiment of a bone screw according to the invention the thread 22 in the distal portion 121 is formed by the thread crest 23 and a thread groove 24 for at least one convolution, preferably at least two, in particular two convolutions of the thread.

In the embodiment of FIG. 4A a portion 122 in which the thread 22 is formed by the thread crest 23 only extends from the proximal end 25 to at least three quarters of the length of the screw, in particular to three quarters of the length of the screw.

In a preferred embodiment of a bone screw according to the invention the said portion 122 comprises at least one convolution, preferably from two to six convolutions, in particular from three to four convolutions of the thread.

As shown in the embodiment of FIG. 4A, the screw in said portion 122 is open to the inside 29 in order to allow bone to grow into the inner parts of the screw, once the screw has been screwed into a patient's bone. Furthermore, the screw is axially compressible in the direction of the longitudinal axis.

Again it is to be noted that the above described embodiment of the screw is devoid of a screw body as generally referred to in the prior art. It is further to be noted, that in the above embodiment, as shown in FIG. 4A, the inner engagement contour 28 is formed entirely in the inner wall of the thread crest 23.

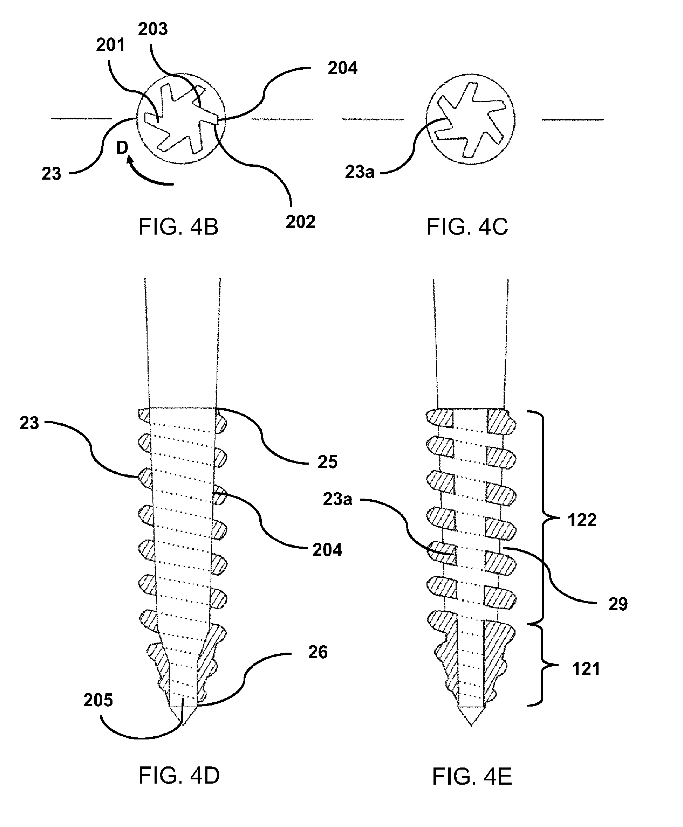

FIG. 4B and FIG. 4C show top views of the screw of FIG. 4A, in order to illustrate the design of the inner engagement contour. The outer circular line of FIG. 4B (as well as of FIG. 4C) represent the thread crest 23 of the screw. The inner contour line in FIG. 4B and FIG. 4B indicates the recesses 201 in the inner wall of the thread crest 23. In the embodiment of FIGS. 4A-E the recesses 201 of the inner engagement contour 28 are formed in the inner wall of the thread crest 23 only.

In particular, in FIG. 4B and FIG. 4C the inner engagement contour 28 is represented in a cross-section of the screw 21, at right angles to the longitudinal axis L, and generally comprises from three to eight recesses 201, in FIG. 4B and 4C in particular six recesses 201, which are all inclined in the direction of rotation D for screwing-in of the screw. Each recess 201 comprises a leading flank 202 and a trailing flank 203 and optionally a base 204. The leading flank 202 is shorter than the trailing flank 203 and thus, at a certain radial distance from the longitudinal axis L, the flanks will intersect or converge at an angle to close the recess 201. The leading flank 202 is for example inclined at an angle of up to 10 degrees and in FIGS. 4B and 4C in particular at an angle of about 5 degrees. Optionally the recess 201 may be closed by a base 204, which at a certain radial distance from the longitudinal axis L is connecting the flanks 202, 203, to form a bottom of the recess 201.

In the embodiment of FIG. 4B and 4C the inner engagement contour 28, i.e. the recesses 201 together substantially resemble a sawtooth-like profile.

Further suitable engagement contours are described in EP-A-0674880. In particular the engagement contours represented there in FIGS. 3-6, 9, 11, 13 and 15, with leading and trailing flanks (indicated as 24 and 25 in EP-A-0674880) directly intersecting (or converging) and thus closing the recesses (indicated as 23 there), as well as those represented in FIGS. 7, 8, 10, 12, 14 and 16 of EP-A-0674880, with leading and trailing flanks (indicated as 24 and 25 there) closed by a base (indicated as 27 there) connecting the flanks at certain radial distance from the longitudinal axis (indicated as 14 therein), thus forming a bottom of the recesses (indicated as 23 therein), are considered suitable engagement contours. Within the meaning of the present invention, all embodiments of the engagement contour shown in EP-A-0674880 substantially resemble a sawtooth-like profile.

As a consequence of the the above described design, the inner engagement contour of the bone screw of the present invention is rotationally symmetrical about the longitudinal axis L at fixed rotational angles, and is asymmetrical with respect to a diametrical plane including the longitudinal axis L. Rotational symmetry of order n, also called n-fold rotational symmetry, or discrete rotational symmetry of the n^{th} order, with respect to a an axis is defined in that rotation by an angle of 360°/n does not change the object (e.g. for a C₄ symmetrie, i.e. n being 4 a rotation by an angle of 90° does not change the object).

FIGS. 4D and 4E show the screw of FIG. 4A in two longitudinal sections at different rotational angles shown in FIGS. 4B and 4C. Due to the different rotational angles of the longitudinal sections, FIG. 4D basically shows a section through two recesses 201 in the inner wall of the thread crest 23, i.e. in a position where the recesses 201 are closed by a base 204 at a certain maximum radial distance from the longitudinal axis L. FIG. 4E however shows a section where there are basically no recesses in the inner wall of the thread crest, i.e. where the remaining parts 23 a of the inner wall of the thread crest are closest to the longitudinal axis L. With reference to these remaining parts of the inner wall of the thread crest an inner channel of the screw is defined. In said inner channel a cylindrical shaft (as support for the blades) of a screwdriver can be inserted, whereas the blade part of the screwdriver (provided on said cylindrical shaft) is engaging with the recesses of the engagement contour.

In the embodiment of FIG. 4A-E the inner engagement contour 28 extends from the proximal end 25 of the screw 21 substantially to the distal end 26 of the screw 21. Wherein in the present context the term "substantially to the distal end of the screw" refers to at least about 80% of the length of the screw, preferably at least about 85% of the length of the screw and in particular about seven eighth of the length of the screw. In another embodiment (not shown in the Figures) the inner engagement contour preferably extends through the entire screw from the proximal end to the distal end of the screw.

Accordingly, the screw as shown in FIGS. 4A-E, in the portion where the screw is formed by the thread crest 23 only, is open to the inside in order to allow bone to grow into the inner parts of the screw, once the screw has been screwed into a bone. The opening 29 is provided at a location, where in a conventional screw a thread groove would be provided, i.e. adjacent to the thread crest 23 and extending about the longitudinal axis L of the screw 21, and with the same direction of rotation D as the external thread.

In FIGS. 4D and 4E a shaft of a screwdriver inserted into the screw through the proximal end 25 is indicated, in particular with the tip 214 of the shaft 211 (see FIG. 2) extending through an opening 205 at the distal end 26 of the screw 21. Thus, with the screwdriver inserted, the blades 213 (see FIG. 2) of the screwdriver are partly visible from the outside through the opening 19 of the screw, whereas when covered by the external thread 23, the said blades are within the recesses 201 and thus not visible from the outside.

It is to be noted, that in a further conceivable embodiment of the invention, upon screwing in of the screw and provided that the blades 213 (see FIG. 2) of the screwdriver 210 are engaging with the bases 204 of the recesses 201, the forces exerted through compression and friction may (at least partly) break open the bases 204 and thus expose the blades 213 to the outside. Upon retracting the screwdriver, the srew 23 may thus provide additional openings to the inside of the screw, where the wall of the recesses 201 to the outside of the screw (i.e. at the bases 204) has been removed.

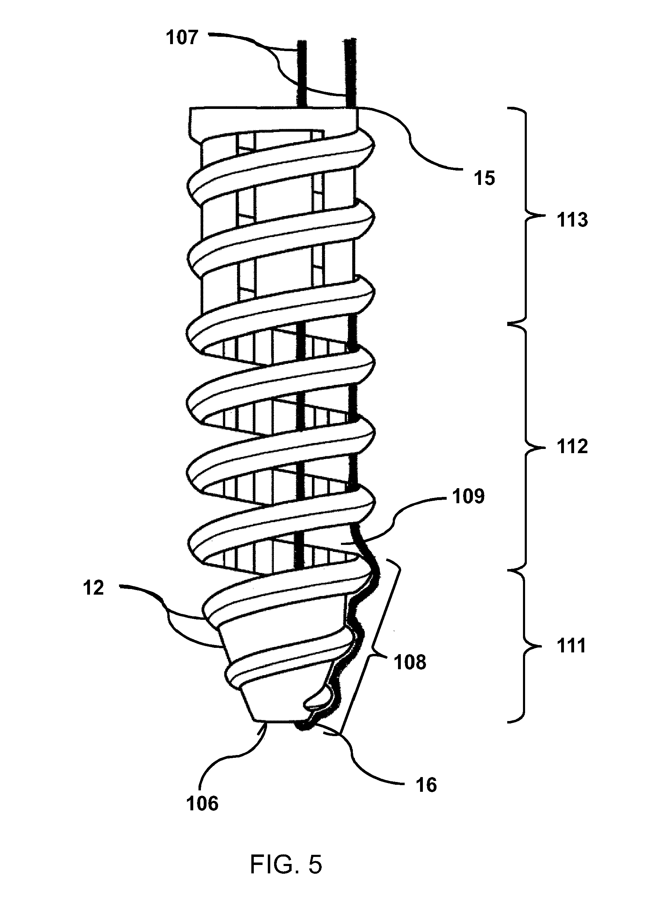

FIG. 5 shows in a perspective side view another embodiment of a bone screw, in particular a suture anchor according to the invention. The bone screw is essentially identical to that one described in FIG. 3A above. The screw 11 (see FIG. 3A) comprises an opening 105 (see FIG. 3D) through the distal end face 106 of the screw. The suture anchor further comprises a suture 107 affixed to the distal end 16 of the screw 11.

In the embodiment shown in FIG. 5 the suture 107 enters the screw at the proximal end 15, extends along the inside of the screw, and passes through an opening 105 (see FIG. 3D) in the distal end face 106 of the screw. The suture then extends along a portion 108 of the outer contour of the screw (closely conforming to the thread grooves 14 and thread crests 13 of the distal portion 111 of the screw, see FIG. 3A). The suture 107 then passes into the inside of the screw through an opening 109 in the portion 112 of the screw 11 in which the thread 12 is formed by the thread crest 13 only, and finally the suture 107 extends again along the inside of the screw over the entire length of the screw out of the proximal end 15 of the screw.

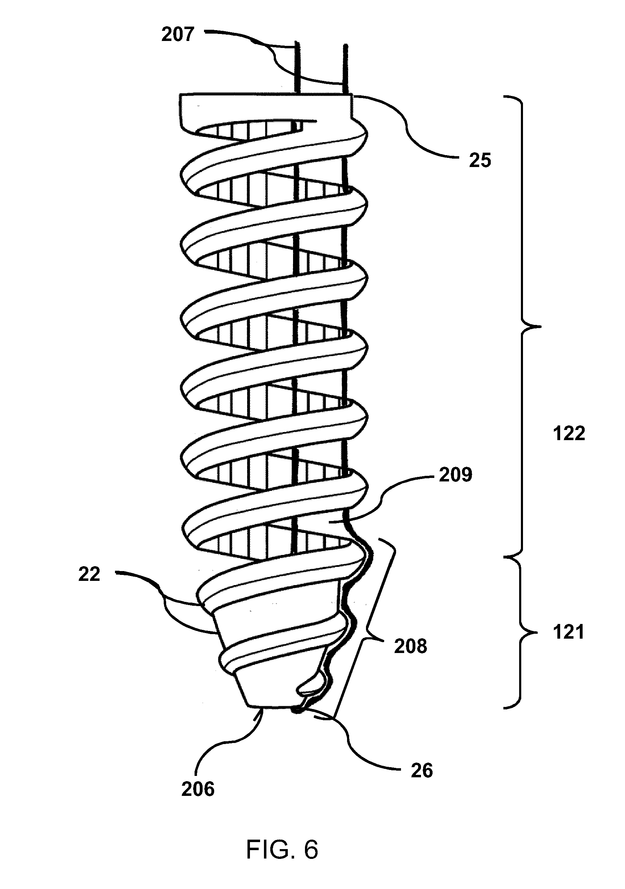

FIG. 6 shows in a perspective side view another embodiment of a bone screw, in particular a suture anchor according to the invention. The bone screw is essentially identical to that one described in FIG. 4A above. The screw 21 (see FIG. 4A) comprises an opening 205 (see FIG. 4D) through the distal end face 206 of the screw. The suture anchor further comprises a suture 207 affixed to the distal end 26 of the screw 21.

In the embodiment shown in FIG. 6 the suture 207 enters the screw at the proximal end 25, extends along the inside of the screw, and passes through an opening 205 in the distal end face 206 of the screw. The suture then extends along a portion 208 of the outer contour of the screw (closely conforming to the thread grooves 24 and thread crests 23 of the distal portion 121 of the screw, see FIG. 4A). The suture 207 then passes into the inside of the screw through an opening 209 in the portion 122 of the screw 21 in which the thread 22 is formed by the thread crest 23 only, and finally the suture 207 extends again along the inside of the screw over the entire length of the screw out of the proximal end 25 of the screw.

In another embodiment not shown in the Figures, the suture 107; 207 may first extend along the outer contour of the screw (either the screw of FIG. 5 or the screw FIG. 6) along the entire length of the screw from the proximal end 15; 25 to the distal end 16; 26 of the screw 11; 21 and then, on the same or on the other side of the screw, may extend back to and beyond the proximal end 15; 25 of the screw.

In yet another embodiment also not shown in the Figures, the suture 107; 207 may first extend along the inside of the screw (either the screw of FIG. 5 or the screw FIG. 6) along the entire length of the screw, may pass through an opening 105; 205 in the distal end face 106; 206 of the screw 11; 21, and may then extend along the outer contour of the screw 11; 21 over the entire length of the screw from the distal end 16; 26 beyond the proximal end 15; 25 of the screw.

### INDUSTRIAL APPLICABILITY OF THE INVENTION

In one aspect the screw of the present invention is suitable for use as an interference screw, i.e. a screw wich is commonly used for fixation of the anterior cruciate ligament of the knee. The interference screw thereby is used as a clamping device for the ligament. As metal screws carry the risk of immediate or late postoperative complications, such as tendon-graft lacerations or difficulties during hardware removal, and as radiological follow-up studies are often distorted, non-metallic interference screws, in particular biodegradable interference screws, are generally preferred.

In another aspect, the screw of the present invention is suitable for use as a suture anchor, i.e. a fixation device for fixing implants, tendons or ligaments to bone. A suture anchor generally comprises an anchor, which is inserted into the bone, and an eyelet, which is a hole or a loop in the anchor through which the suture passes. The suture as well as the anchor may be made of a biocompatible non-absorbable material or of an absorbable (i.e. biodegradable) material.

## Claims

1. A bone screw (1; 11; 21) comprising:
an external thread (2; 12; 22) extending about a longitudinal axis (L) of the screw, the external thread (2; 12; 22) comprising a thread crest (3; 13; 23);
an inner engagement contour (8; 18; 28) for receiving a complementary engagement contour (211) of a screwdriver (210);
wherein in at least a portion of the screw (1; 11; 21) the thread (2; 12; 22) is formed by the thread crest (3; 13; 23) only.

2. Bone screw according to claim 1, wherein the screw further comprises one or more inner elongated ribs (7; 17) extending from a proximal end (5; 15) towards a distal end (6; 16) of the screw (1; 11) and connecting the thread crest (3; 13) over the length of the elongated ribs (7; 17).

3. Bone screw according to claim 2, wherein the one or more inner elongated ribs (7; 17) are extending from the proximal end (5; 15) over at least one third of the length of the screw.

4. Bone screw according to claim 3 further comprising an intermediate portion (112), which extends from the end of the portion (113) comprising the one or more inner elongated ribs (17) to three quarters of the length of the screw, wherein in said intermediate portion (112) the thread (12) is formed by the thread crest (13) only.

5. Bone screw according to claim 1, wherein the portion (122) of the screw (1; 11; 21) in which the thread (2; 12; 22) is formed by the thread crest (3; 13; 23) only, extends from the proximal end (5; 15; 25) of the screw (1; 11; 21) towards the distal end (6; 16; 26) of the screw over at least three quarters of the length of the screw (1; 11; 21).

6. Bone screw according to any of the preceding claims further comprising a distal portion (101; 111; 121) extending from the distal end (6; 16; 26) towards the proximal end (5; 15; 25) of the screw over one quarter of the length of the screw (1; 11; 21), wherein the external thread (2; 12; 22) comprises a thread crest (3; 13; 23) and a thread groove (4; 14; 24).

7. Bone screw according to any of the preceding claims, wherein the screw is tapered.

8. Bone screw according to any of the preceding claims, wherein the inner engagement contour (8; 18; 28) extends from the proximal end (5; 15; 25) of the screw (1; 11; 21) substantially to the distal end (6; 16; 26) of the screw (1; 11; 21).

9. Bone screw according to any of the preceding claims, wherein the inner engagement contour (8; 18; 28) is at least partly formed in the inner wall of the thread crest (3; 13; 23) and comprises two or more recesses (201) which are inclined in a direction of rotation (D) for screwing-in of the screw (1; 11; 21), each recess (201) comprising a leading flank (202) and a trailing flank (203), with the leading flank (202) being shorter than the trailing flank (203).

10. Bone screw according to claim 9, wherein the inner engagement contour (8; 18; 28) comprises three to eight recesses (201), in particular six recesses (201).

11. Bone screw according to any of the preceding claims, wherein the screw (1; 11; 21) further comprises an opening (105; 205) through the distal end face (106; 206) of the screw.

12. A suture anchor comprising a bone screw according to any of the preceding claims and further comprising a suture (107; 207) affixed to the distal end (16; 26) of the screw (11; 21).

13. A suture anchor according to claim 12, wherein
(A) the suture (107; 207) first extends along the outer contour of the screw (11; 21) along the entire length of the screw from the proximal end (15; 25) to the distal end (16; 26) of the screw and then on the same or on the other side of the screw extends back to and beyond the proximal end (15; 25) of the screw; or
(B) the suture (107; 207) first extends along the inside of the screw (11; 21) along the entire length of the screw, passes through an opening (105; 205) in the distal end face (106; 206) of the screw, and then extends along the outer contour of the screw (11; 21) over the entire length of the screw from the distal end (16; 26) to and beyond the proximal end (15; 25) of the screw; or
(C) the suture (107; 207) extends along the inside of the screw (11; 21) along the entire length of the screw, passes through an opening (105; 205) in the distal end face (106; 206) of the screw, then extends along a portion (108; 208) of the outer contour of the screw (11; 21) over the thread crest (13; 23) and thread groove (14; 24) of the external thread (12; 22) in the distal portion (111; 121), then passes into the inside of the screw through an opening (109; 209) in the portion of the screw (11; 21) in which the thread (12; 22) is formed by the thread crest (13; 23) only, and finally extends again along the inside of the screw over the entire length of the screw out of the proximal end (15; 25) of the screw.

14. A method for fixing a screw in a bone, wherein in a first step a screw according to any one of claims 1-11 is screwed into a bone by means of a screwdriver.

15. A method for fixing a suture anchor in a bone, wherein in a first step a suture anchor according to claim 12 or 13 is screwed into a bone by means of a screwdriver (210), and wherein in a second step the distal end (16; 26) of the screw (11; 21) is tilted and/or at least partly compressed by means of pulling the suture (107; 207) extending beyond the proximal end (15; 25) of the screw (11; 21).
